# EUROPEAN PATENT SPECIFICATION

(11) **EP 1 238 058 B1**
(45) Date of publication and mention of the grant of the patent: **03.10.2007**
(21) Application number: 00957579.6
(22) Date of filing: 17.08.2000
(51) Int. Cl.: C12N 5/06, A61K 38/18

(54) **USE OF COLOSTRININ, CONSTITUENT PEPTIDES AND ANALOGS THEREOF TO PROMOTE NEURAL CELL DIFFERENTIATION**
VERWENDUNG VON COLOSTRININ, DESSEN PEPTIDBESTANDTEILE UND DEREN ANALOGA ZUR FÖRDERUNG DER NEURONALEN ZELLDIFFERENZIERUNG
UTILISATION DE LA COLOSTRININE, DE SES PEPTIDES CONSTITUTIFS ET ANALOGUES POUR ACTIVER UNE DIFFERENCIATION DE CELLULES NERVEUSES

(30) Priority: 17.08.1999 US 149633 P
(43) Date of publication of application: 11.09.2002
(73) Proprietor: THE UNIVERSITY OF TEXAS SYSTEM, Austin, Texas 78701-2981 (US)
(72) Inventor: BOLDOGH, Istvan, Galveston, TX 77550 (US); HUGHES, Thomas, K., Jr., Galveston, TX 77554 (US); STANTON, G. JOHN, Texas City, TX 77591 (US)
(74) Representative: Curtis, Philip Anthony
(86) International application number: PCT/US2000/022774
(87) International publication number: WO 2001/012651

(56) References cited:
- WO-A1-98/14473
- POPIK P ET AL: "COLOSTRININ, A POLYPEPTIDE ISOLATED FROM EARLY MILK, FACILITATES LEARNING AND MEMORY IN RATS" PHARMACOLOGY BIOCHEMISTRY AND BEHAVIOR, ELSEVIER, US, vol. 64, no. 1, 1999, pages 183-189, XP002938310 ISSN: 0091-3057
- COSGAYA J M ET AL: "Neuronal differentiation of PC12 cells induced by engrailed homeodomain is DNA-binding specific and independent of MAP kinases." JOURNAL OF CELL SCIENCE. ENGLAND AUG 1998, vol. 111 ( Pt 16), August 1998 (1998-08), pages 2377-2384, XP002217467 ISSN: 0021-9533
- JANUSZ M AND LISOWSKI J: "Proline-rich polypeptide (PRP) - an immunomodulatory peptide from ovine colostrum" ARCHIVUM IMMUNOLOGIAE ET THERAPIAE EXPERIMENTALIS, POLISH ACADEMY OF SCIENCES, WROCLAW, PL, vol. 41, no. 5-6, 1993, pages 275-279, XP002055885 ISSN: 0004-069X

### SEQUENCE LISTING

<110> THE UNIVERSITY OF TEXAS SYSTEM
   BOLDOGH, Istvan
<120> USE OF COLOSTRININ, CONSTITUENT PEPTIDES THEREOF, AND ANALOGS THEREOF TO PROMOTE NEURAL CELL DIFFERENTIATION
<130> 265.00240201
<140> Unassigned
   <141> 2000-08-17
<150> 60/149,633
   <151> 1999-08-17
<160> 34
<170> PatentIn Ver. 2.1
<210> 1
   <211> 7
   <212> PRT
   <213> Artificial Sequence
<220>
   <223> Description of Artificial Sequence: synthetic peptide
<400> 1
   Met Gln Pro Pro Pro Leu Pro
    1               5
<210> 2
   <211> 17
   <212> PRT
   <213> Artificial Sequence
<220>
   <223> Description of Artificial Sequence: synthetic peptide
<400> 2
   Leu Gln Thr Pro Gln Pro Leu Leu Gln Val Met Met Glu Pro Gln Gly
    1               5                  10                  15
   Asp
<210> 3
   <211> 18
   <212> PRT
   <213> Artificial Sequence
<220>
   <223> Description of Artificial Sequence: synthetic peptide
<400> 3
   Asp Gln Pro Pro Asp Val Glu Lys Pro Asp Leu Gln Pro Phe Gln Val
    1               5                  10                  15
   Gln Ser
<210> 4
   <211> 12
   <212> PRT
   <213> Artificial Sequence
<220>
   <223> Description of Artificial Sequence: synthetic peptide
<400> 4
   Leu Phe Phe Phe Leu Pro Val Val Asn Val Leu Pro
    1               5                  10
<210> 5
   <211> 15
   <212> PRT
   <213> Artificial Sequence
<220>
   <223> Description of Artificial Sequence: synthetic peptide
<400> 5
   Asp Leu Glu Met Pro Val Leu Pro Val Glu Pro Phe Pro Phe Val
    1               5                  10                  15
<210> 6
   <211> 11
   <212> PRT
   <213> Artificial Sequence
<220>
   <223> Description of Artificial Sequence: synthetic peptide
<400> 6
   Met Pro Gln Asn Phe Tyr Lys Leu Pro Gln Met
    1               5                  10
<210> 7
   <211> 15
   <212> PRT
   <213> Artificial Sequence
<220>
   <223> Description of Artificial Sequence: synthetic peptide
<400> 7
   Val Leu Glu Met Lys Phe Pro Pro Pro Pro Gln Glu Thr Val Thr
    1               5                  10                  15
<210> 8
   <211> 15
   <212> PRT
   <213> Artificial Sequence
<220>
   <223> Description of Artificial Sequence: synthetic peptide
<400> 8
   Leu Lys Pro Phe Pro Lys Leu Lys Val Glu Val Phe Pro Phe Pro
    1               5                  10                  15
<210> 9
   <211> 5
   <212> PRT
   <213> Artificial Sequence
<220>
   <223> Description of Artificial Sequence: synthetic peptide
<400> 9
   Val Val Met Glu Val
    1               5
<210> 10
   <211> 4
   <212> PRT
   <213> Artificial Sequence
<220>
   <223> Description of Artificial Sequence: synthetic peptide
<400> 10
   Ser Glu Gln Pro
    1
<210> 11
   <211> 3
   <212> PRT
   <213> Artificial Sequence
<220>
   <223> Description of Artificial Sequence: synthetic peptide
<400> 11
   Asp Lys Glu
    1
<210> 12
   <211> 5
   <212> PRT
   <213> Artificial Sequence
<220>
   <223> Description of Artificial Sequence: synthetic peptide
<400> 12
   Phe Pro Pro Pro Lys
    1               5
<210> 13
   <211> 6
   <212> PRT
   <213> Artificial Sequence
<220>
   <223> Description of Artificial Sequence: synthetic peptide
<400> 13
   Asp Ser Gln Pro Pro Val
   1 5
<210> 14
   <211> 7
   <212> PRT
   <213> Artificial Sequence
<220>
   <223> Description of Artificial Sequence: synthetic peptide
<400> 14
   Asp Pro Pro Pro Pro Gln Ser
   1 5
<210> 15
   <211> 5
   <212> PRT
   <213> Artificial Sequence
<220>
   <223> Description of Artificial Sequence: synthetic peptide
<400> 15
   Ser Glu Glu Met Pro
   1 5
<210> 16
   <211> 7
   <212> PRT
   <213> Artificial Sequence
<220>
   <223> Description of Artificial Sequence: synthetic peptide
<400> 16
   Lys Tyr Lys Leu Gln Pro Glu
   1 5
<210> 17
   <211> 7
   <212> PRT
   <213> Artificial Sequence
<220>
   <223> Description of Artificial Sequence: synthetic peptide
<400> 17
   Val Leu Pro Pro Asn Val Gly
   1 5
<210> 18
   <211> 10
   <212> PRT
   <213> Artificial Sequence
<220>
   <223> Description of Artificial Sequence: synthetic peptide
<400> 18
   Val Tyr Pro Phe Thr Gly Pro Ile Pro Asn
   1 5 10
<210> 19
   <211> 9
   <212> PRT
   <213> Artificial Sequence
<220>
   <223> Description of Artificial Sequence: synthetic peptide
<400> 19
   Ser Leu Pro Gln Asn Ile Leu Pro Leu
   1 5
<210> 20
   <211> 10
   <212> PRT
   <213> Artificial Sequence
<220>
   <223> Description of Artificial Sequence: synthetic peptide
<400> 20
   Thr Gln Thr Pro Val Val Val Pro Pro Phe
   1 5 10
<210> 21
   <211> 18
   <212> PRT
   <213> Artificial Sequence
<220>
   <223> Description of Artificial Sequence: synthetic peptide
<400> 21
   Leu Gln Pro Glu Ile Met Gly Val Pro Lys Val Lys Glu Thr Met Val
    1               5                  10                  15
   Pro Lys
<210> 22
   <211> 18
   <212> PRT
   <213> Artificial Sequence
<220>
   <223> Description of Artificial Sequence: synthetic peptide
<400> 22
   His Lys Glu Met Pro Phe Pro Lys Tyr Pro Val Glu Pro Phe Thr Glu
    1               5                  10                  15
   Ser Gln
<210> 23
   <211> 18
   <212> PRT
   <213> Artificial Sequence
<220>
   <223> Description of Artificial Sequence: synthetic peptide
<400> 23
   Ser Leu Thr Leu Thr Asp Val Glu Lys Leu His Leu Pro Leu Pro Leu
    1               5                  10                  15
   Val Gln
<210> 24
   <211> 7
   <212> PRT
   <213> Artificial Sequence
<220>
   <223> Description of Artificial Sequence: synthetic peptide
<400> 24
   Ser Trp Met His Gln Pro Pro
    1               5
<210> 25
   <211> 10
   <212> PRT
   <213> Artificial Sequence
<220>
   <223> Description of Artificial Sequence: synthetic peptide
<400> 25
   Gln Pro Leu Pro Pro Thr Val Met Phe Pro
    1               5                   10
<210> 26
   <211> 6
   <212> PRT
   <213> Artificial Sequence
<220>
   <223> Description of Artificial Sequence: synthetic peptide
<400> 26
   Pro Gln Ser Val Leu Ser
    1               5
<210> 27
   <211> 22
   <212> PRT
   <213> Artificial Sequence
<220>
   <223> Description of Artificial Sequence: synthetic peptide
<400> 27
   Leu Ser Gln Pro Lys Val Leu Pro Val Pro Gln Lys Ala Val Pro Gln
    1               5                  10                  15
   Arg Asp Met Pro Ile Gln
               20
<210> 28
   <211> 7
   <212> PRT
   <213> Artificial Sequence
<220>
   <223> Description of Artificial Sequence: synthetic peptide
<400> 28
   Ala Phe Leu Leu Tyr Gln Glu
   1 5
<210> 29
   <211> 8
   <212> PRT
   <213> Artificial Sequence
<220>
   <223> Description of Artificial Sequence: synthetic peptide
<400> 29
   Arg Gly Pro Phe Pro Ile Leu Val
   1 5
<210> 30
   <211> 18
   <212> PRT
   <213> Artificial Sequence
<220>
   <223> Description of Artificial Sequence: synthetic peptide
<400> 30
   Ala Thr Phe Asn Arg Tyr Gln Asp Asp His Gly Glu Glu Ile Leu Lys
   1 5 10 15
   Ser Leu
<210> 31
   <211> 9
   <212> PRT
   <213> Artificial Sequence
<220>
   <223> Description of Artificial Sequence: synthetic peptide
<400> 31
   Val Glu Ser Tyr Val Pro Leu Phe Pro
   1 5
<210> 32
   <211> 13
   <212> PRT
   <213> Artificial Sequence
<220>
   <223> Description of Artificial Sequence: synthetic peptide
<400> 32
   Phe Leu Leu Tyr Gln Glu Pro Val Leu Gly Pro Val Arg
   1 5 10
<210> 33
   <211> 3
   <212> PRT
   <213> Artificial Sequence
<220>
   <223> Description of Artificial Sequence: synthetic peptide
<400> 33
   Leu Asn Phe
   1
<210> 34
   <211> 15
   <212> PRT
   <213> Artificial Sequence
<220>
   <223> Description of Artificial Sequence: synthetic peptide
<400> 34
   Met His Gln Pro Pro Gln Pro Leu Pro Pro Thr Val Met Phe Pro
   1 5 10 15

## Description

Colostrum is a component of the milk of mammals during the first few days after birth. Colostrum is a thick yellowish fluid and is the first lacteal secretion post parturition and contains a high concentration of immunogloblins (IgG, IgM, and IgA) and a variety of non-specific proteins. Colostrum also contains various cells such as granular and stromal cells, neutrophils, monocyte/macrophages, and lymphocytes. Colostrum also includes growth factors, hormones, and cytokines. Unlike mature breast milk, colostrum contains low sugar, low iron, but is rich is lipids, proteins, mineral salts, vitamins, and immunoglobins.

Colostrum also includes or contains a proline-rich polypeptide aggregate or complex, which is referred to as colostrinin. One peptide fragment of colostrinin is Val-Glu-Ser-Tyr-Val-Pro-Leu-Phe-Pro (SEQ ID NO:31), which is disclosed in International Publication No. WO-A-98/14473. Colostrinin and this fragment have been identified as useful in the treatment of disorders of the central nervous system, neurological disorders, mental disorders, dementia, neurodegenerative diseases, Alzheimer's disease, motor neurone disease, psychosis, neurosis, chronic disorders of the immune system, diseases with a bacterial and viral aetiology, and acquired immunological deficiencies as set forth in International Publication No. WO-A-98/14473.

Although certain uses for colostrinin have been identified, it would represent an advancement in the art to discover and disclose other uses for colostrinin, or a component thereof, that are not readily ascertainable from the information currently known about colostrinin or its constituents.

The present invention relates to the use of colostrinin, at least one constituent (i.e., component) peptide thereof, at least one active analog thereof (e.g., peptidehaving an N-terminal seqnece equivalent to an N-terminal sequence of at least one of the colostrinin constituent peptides), and combinations thereof, as promoters of neural cell differentiation. These agents can be used in *vitro* or in *vivo,* including internal use in patients, particularly animals (including mammals such as humans).

In a first aspect the present invention provides a method for promoting in *vitro* cell differentiation, the method comprising contacting cells with a neuronal cell regulator selected from the group of colostrinin, a constituent peptide of colostrinin, an active analog of a constituent peptide of colostrinin, and combination thereof, under conditions effective to change the cells in morphology to form neuronal cells, wherein the constituent peptide of colostrinin is selected from the group of SEQ ID NO: 1 through SEQ ID NO: 34, and wherein the active analog comprises a peptide having an amino acid sequence with at least 15 percent proline and having at least 70 percent sequence identity to one or more constituent pepetides of colostrinin, which are selected from the group of SEQ ID NO: 1 through SEQ ID NO: 34. The cells can be present in a cell culture, an organ, a tissue, or an organism. Preferably, the cells are mammalian cells, and more preferably, human cells. The neuronal cell regulator is preferably a constituent peptide of colostrinin, such as those described herein (SEQ ID Nos: 1-34).

In a second aspect, the present invention provides the use of a neuronal cell regulator selected from the group of colostrinin, a constituent peptide of colostrinin, an active analog of a constituent peptide of colostrinin, and combinations thereof, wherein the constituent peptide of colostrinin is selected from the group of SEQ ID NO: 1 through SEQ ID NO: 34; and wherein the active analog comprises a peptide having an amino acid sequence with at least 15 percent proline and having at least 70 percent sequence identify to one or more constituent peptides of colostrinin, which are selected from the group of SEQ ID NO: 1 through SEQ ID NO: 34, in the manufacture of a medicament for promoting neuronal cell differentiation in a patient.

In a third aspect, the present invention provides an in *vitro* method for treating damaged neuronal cells, the method comprising contacting non-functional neuronal cells with a neuronal cell regulator selected from the group of colostrinin, a constituent peptide of colostrinin, an active analog of a constituent peptide of colostrinin, and combinations thereof, under conditions effective to convert the damaged neuronal cells to functional neuronal cells, wherein the constituent peptide of colostrinin is selected from the group of SEQ ID NO: 1 through SEQ ID NO: 34; wherein the active analog comprises a peptide having an amino acid sequence with at least 15 percent proline and having at least 70 percent sequence identity to one or more constituent peptides of colostrinin, which are selected from the group of SEQ ID NO: 1 through SEQ ID NO: 1 through SEQ ID NO: 34, and wherein the non-functional is the result of neurodegeneration.

An in *vivo* method for treating damaged (e.g., non-functional) neural cells in a patient includes administering to the patient a neural cell regulator selected from the group of colostrinin, a constituent peptide thereof, an analog thereof, and combinations thereof, under conditions effective to convert damaged neural cells to functional neural cells.

In a fourth aspect, the present invention provides the use of a neuronal cell regulator selected from the group of colostrinin, a constituent peptide of colostrinin, an active analog of a constituent peptide of colostrinin, and combinations thereof, wherein the constituent peptide of colostrinin is selected from the group of SEQ ID NO: 1 through SEQ ID NO: 34; wherein the active analog comprises a peptide having an amino acid sequence with at least 15 percent proline and having at least 70 percent sequence identity to one or more constituent peptides of colostrinin, which are selected from the group of SEQ ID NO: 1 through SEQ ID NO: 34, in the manufacture of a medicament for treating damaged neuronal cells in a patient.

In a fifth aspect, the present invention provides a method for promoting in *vitro* neuronal cell differentiation, the method comprising contacting pluripotent cells of the nervous system with a neuronal cell regulator selected from the group of colostrinin, a constituent peptide of colostrinin, an active analog of a constituent peptide of colostrinin, and combination thereof, under conditions effective to change the pluripotent cells of the nervous system in morphology to form neuronal cells, wherein the constituent peptide of colostrinin is selected from the group of SEQ ID NO: 1 thorough SEQ ID NO: 34; wherein the active analog comprises a peptide having an amino acid sequence with at least 15 percent proline and having at least 70 percent sequence identity to one or more constituent peptides of colostrinin, which are selected from the group of SEQ ID NO: 1 through SEQ ID NO: 34.

In a sixth aspect, the present invention provides the use of a neuronal cell regulator selected from the group of colostrinin, a constitute peptide of colostrinin, an active analog of a constituent peptide of colostrinin, and combinations thereof, wherein the constituent peptide of colostrinin is selected from the group of SEQ ID NO: 1 to SEQ ID NO: 34; and wherein the active analog comprises a peptide having an amino acid sequence with at least 15 percent proline and having at least 70 percent sequence identity to one or more constituent peptides of colostrinin, which are selected from the group of SEQ ID NO: 1 through SEQ ID NO: 34, in the manufacture of a medicament for promoting differentiation of pluripotent cells of the nervous system to form neuronal cells in a patient.

As used herein, "neural" and "nerve-like" are used interchangeably. Such cells have morphologies resembling nerve cells. For example, a central body with neurite outgrowth. As used herein, non-functional neural cells are those that do not transmit information by, e.g. acetylcholine, but morphologically resemble nerve cells, and functional neural cells are those that do transmit information using mediators such as acetylcholine and morphologically resemble nerve cells.

As used herein, "a" or "an" means one or more, such that combinations of active agents (i.e., active immunological regulators or blood cell differentiation promoters), for example, can be used in the compositions and methods of the invention. Thus, a composition that includes "a" polypeptide refers to a composition that includes one or more polypeptides.

"Amino acid" is used herein to refer to a chemical compound with the general formula: NH₂-CRH-COOH, where R, the side chain, is H or an organic group. Where R is organic, R can vary and is either polar or nonpolar (i.e., hydrophobic). The amino acids of this invention can be naturally occurring or synthetic (often referred to as nonproteinogenic). As used herein, an organic group is a hydrocarbon group that is classified as an aliphatic group, a cyclic group or combination of aliphatic and cyclic groups. The term "aliphatic group" means a saturated or unsaturated linear or branched hydrocarbon group. This term is used to encompass alkyl, alkenyl, and alkynyl groups, for example. The term "cyclic group" means a closed ring hydrocarbon group that is classified as an alicyclic group, aromatic group, or heterocyclic group. The term "alicyclic group" means a cyclic hydrocarbon group having properties resembling those of aliphatic groups. The term "aromatic group" refers to mono- or polycyclic aromatic hydrocarbon groups. As used herein, an organic group can be substituted or unsubstituted.

The terms "polypeptide" and "peptide" are used interchangeably herein to refer to a polymer of amino acids. These terms do not connote a specific length of a polymer of amino acids. Thus, for example, the terms oligopeptide, protein, and enzyme are included within the definition of polypeptide or peptide, whether produced using recombinant techniques, chemical or enzymatic synthesis, or naturally occurring. This term also includes polypeptides that have been modified or derivatized, such as by glycosylation, acetylation, phosphorylation, and the like.

The following abbreviations are used throughout the application:

| | |
|---|---|
| A = Ala=Alanine | T = Thr = Threonine |
| V = Val = Valine | C = Cys = Cysteine |
| L = Leu = Leucine | Y = Tyr = Tyrosine |
| I = De = Isoleucine | N = Asn = Asparagine |
| P = Pro = Proline | Q = Gln = Glutamine |
| F = Phe = Phenylalanine | D = Asp = Aspartic Acid |
| W = Trp = Tryptophan | E = Glu = Glutamic Acid |
| M = Met = Methionine | K = Lys = Lysine |
| G = Gly = Glycine | R = Arg = Arginine |
| S = Ser = Serine | H = His = Histidine |

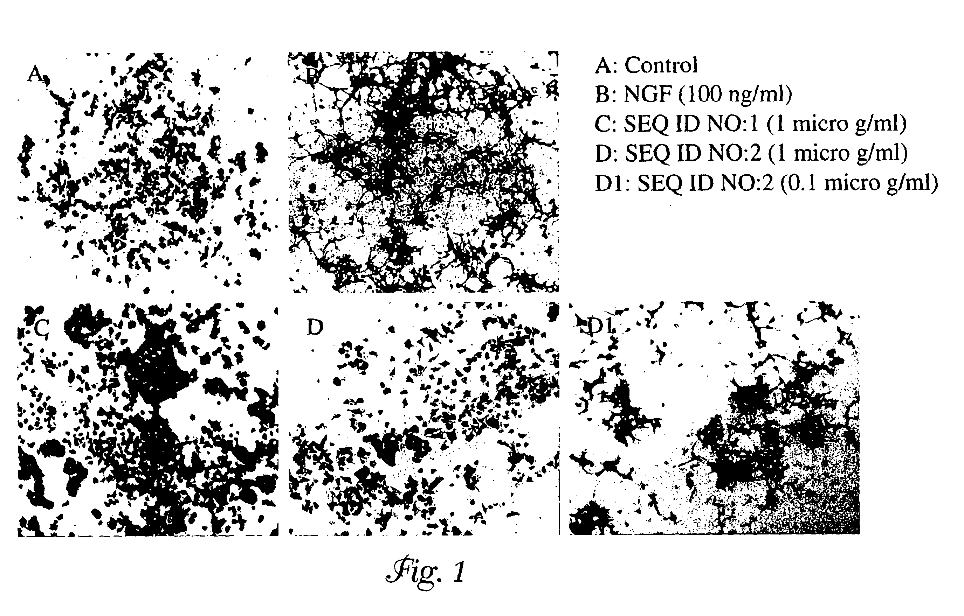

**Figure 1.** Effect of NGF and constituent peptides of colostrinin on morphology of PC12 cells. 3 x 10⁴ cells per well were seeded in 24 x well plates and 24 hours (h) later cells were treated with NGF or colostrum, colostrinin, or its constitutent peptides, as described in the Examples Section. Six days after treatment, cells were fixed in formaldehyde and stained to visualize morphological changes of cells. Mock-treated cells (A), NGF-treated cells (B). Lower panel demonstrate typical morphological changes of PC 12 cells after exposure to SEQ ID NO:1 (C) or SEQ ID NO:2 (D, D1).

The inventors have found that colostrinin, at least one constituent peptide thereof, and/or at least one active analog thereof (e.g., a peptide having an N-terminal sequence equivalent to an N-terminal sequence of at least one of the colostrinin constituent peptides) can be used as neural cell regulators. Such regulators promote the differentiation of cells (e.g., pluripotent cells) such that there is a change in morphology to form neural cells (which can be present in tissues and organs such as brain or ganglion). This can occur *in vitro* or *in vivo*, including internally in animals (including mammals such as humans). These regulators can also convert damaged (e.g., nonfunctional) neural cells to functional neural cells.

Such neural cell regulators are referred to herein as "active agents." Significantly, such agents can be administered alone or in various combinations to a patient (e.g., animals including humans) as a medication or dietary (e.g., nutrient) supplement in a dose sufficient to cause nerve cell increase throughout the patient's body, in a specific tissue site, or in a collection of tissues (organs).

The differentiation process of cells in the nervous system is regulated by the action of differentiation and growth factors including NGF. For example, NGF binding to its receptor tyrosine kinase, TrkA, initiates various molecular interactions including tyrosine phosphorylation of proteins and the action of the Ras/Raf/MEK/MAPK pathway (Chao et al., Cell, 68, 995-997 (1992); and Marshall et al., Cell, 80, 179 -185 (1995)). NGF induces the production of reactive nitric oxide (NO), and NO is required for NGF-induced cytostasis and differentiation (Peunova et al., Nature, 375, 68-73 (1995)), suggesting that free radical molecules may exert a regulatory role in certain types of cellular differentiation.

It is important to promote nerve cell differentiation (i.e., promote differentiation of cells to form neural cells) and/or conversion of damaged nerve cells where there has been significant damage to nerve cells that can occur in a wide variety of situations. The active agents described herein can be used individually, in various combinations, or combined with other previously known or newly invented pharmacological agents. The promotion of nerve cell differentiation responses can be taken advantage of, for example, in cell, tissue, or organ regeneration, repair, and replacement.

In preferred embodiments, the present invention provides methods for promoting neural cell differentiation (i.e., differentiation of cells to form neural cells) and converting nonfunctional neural cells to functional neural cells. Whether it be *in vivo* or *in vitro,* these methods involve monitoring the level of increase in functional nerve cells and/or changes in the morphology of cells formed using phenotypic markers as disclosed by Fillmore et al., J. Neurosci. Res., 31, 662-669 (1992) and Levi et al., Mol. Neurobiol., 2, 201-226 (1988). Specific *in vitro* methods are described in the Examples Section.

Colostrinin is composed of peptides, the aggregate of which has a molecular weight range between about 5.8 to about 26 kiloDaltons (kDa) determined by polyacrylamide gel electrophoresis. It has a greater concentration of proline than any other amino acid. Ovine colostrinin has been found to have a molecular weight of about 18 kDa and includes three non-covalently linked subunits having a molecular weight of about 6 kDa and has about 22 wt-% proline. Ovine colostrinin has also been shown to contain the following number of residues per subunit: lysine - 2; histidine - 1; arginine - 0; aspartic acid - 2; threonine - 4; serine - 3; glutamic acid - 6; proline - 11; glycine - 2; alanine - 0; valine - 5; methionine - 2; isoleucine - 2; leucine - 6; tyrosine - 1; phenylalanine - 3; and cysteine - 0.

Colostrinin has been found to include a number of peptides ranging from 3 amino acids to 22 amino acids or more. These can be obtained by various known techniques, including isolation and purification involving eletrophoresis and synthetic techniques. The specific method of obtaining colostrinin and SEQ ID NO:31 is described in International Publication No. WO-A-98/14473. Using HPLC and Edelman Degradation, over 30 constituent peptides of colostrinin have been identified, which can be classified into several groups: (A) those of unknown precursor; (B) those having a β-casein homologue precursor; (C) those having a β-casein precursor; and (D) those having an annexin precursor. These peptides are described in International Patent Application PCT/GB00/02128, filed June 2, 2000, claiming priority to June 2, 1999, and can be synthesized according to the general method described in the Examples Section. These peptides (i.e., constituent peptides of colostrinin), which can be derived from colostrinin or chemically synthesized, include: MQPPPLP (SEQ ID NO:1); LQTPQPLLQVMMEPQGD (SEQ ID NO:2); DQPPDVEKPDLQPFQVQS (SEQ ID NO:3); LFFFLPWNVLP (SEQ ID NO:4); DLEMPVLPVEPFPFV (SEQ ID NO:5); MPQNFYKLPQM (SEQ ID NO:6); VLEMKFPPPPQETVT (SEQ ID NO:7); LKPFPKLKVEVFPFP (SEQ ID NO:8); VVMEV (SEQ ID NO:9); SEQP (SEQ ID NO: 10); DKE (SEQ ID NO:11); FPPPK (SEQ ID NO:12); DSQPPV (SEQ ID NO:13); DPPPPQS (SEQ ID NO:14); SEEMP (SEQ ID NO:15); KYKLQPE (SEQ ID NO:16); VLPPNVG (SEQ ID NO:17); VYPFTGPIPN (SEQ ID NO: 18); SLPQNILPL (SEQ ID NO:19); TQTPVVVPPF (SEQ ID NO:20); LQPEIMGVPKVKETMVPK (SEQ ID NO:21); HKEMPFPKYPVEPFTESQ (SEQ ID NO:22); SLTLTDVEKLHLPLPLVQ (SEQ ID NO:23); SWMHQPP (SEQ ID NO:24); QPLPPTVMFP (SEQ ID NO:25); PQSVLS (SEQ ID NO:26); LSQPKVLPVPQKAVPQRDMPIQ (SEQ ID NO:27); AFLLYQE (SEQ ID NO:28); RGPFPILV (SEQ ID NO:29); ATFNRYQDDHGEEILKSL (SEQ ID NO:30); VESYVPLFP (SEQ ID NO:31); FLLYQEPVLGPVR (SEQ ID NO:32); LNF (SEQ ID NO:33); and MHQPPQPLPPTVMFP (SEQ ID NO:34). These can be classified as follows: (A) those of unknown precursor include SEQ ID NOs:2, 6, 7, 8, 10, 11, 14, and 33; (B) those having a β-casein homologue precursor include SEQ ID NOs:1, 3, 4, 5, 9, 12, 13, 15, 16, 17, and 31; (C) those having a β-casein precursor include SEQ ID NOs:18 (casein amino acids 74-83), 19 (casein amino acids 84-92), 20 (casein amino acids 93-102), 21 (casein amino acids 103-120), 22 (casein amino acids 121-138), 23 (casein amino acids 139-156), 24 (casein amino acids 157-163), 25 (casein amino acids 164-173), 26 (casein amino acids 174-179), 27 (casein amino acids 180-201), 28 (casein amino acids 202-208), 29 (casein amino acids 214-222), 32 (casein amino acids 203-214), and 34 (casein amino acids 159-173); and (D) those having an annexin precursor include SEQ ID NO:30 (annexin amino acids 203-220).

A preferred group of such peptides includes: MQPPPLP (SEQ ID NO:1); LQTPQPLLQVMMEPQGD (SEQ ID NO:2); DQPPDVEKPDLQPFQVQS (SEQ ID NO:3); LFFFLPVVNVLP (SEQ ID NO:4); DLEMPVLPVEPFPFV (SEQ ID NO:5); MPQNFYKLPQM (SEQ ID NO:6); VLEMKFPPPPQETVT (SEQ ID NO:7); LKPFPKLKVEVFPFP (SEQ ID NO:8); and combinations thereof.

The polypeptides of SEQ ID NOs:1-34 can be in their free acid form or they can be amidated at the C-terminal carboxylate group. The present invention also includes analogs of the polypeptides of SEQ ID NOs:1-34, which includes polypeptides having structural similarity with SEQ ID NOs:1-34. These peptides can also form a part of a larger peptide. An "analog" of a polypeptide includes at least a portion of the polypeptide, wherein the portion contains deletions or additions of one or more contiguous or noncontiguous amino acids, or containing one or more amino acid substitutions. An "analog" can thus include additional amino acids at one or both of the terminii of the polypeptides listed above. Substitutes for an amino acid in the polypeptides of the invention are preferably conservative substitutions, which are selected from other members of the class to which the amino acid belongs. For example, it is well-known in the art of protein biochemistry that an amino acid belonging to a grouping of amino acids having a particular size or characteristic (such as charge, hydrophobicity and hydrophilicity) can generally be substituted for another amino acid without substantially altering the structure of a polypeptide.

For the purposes of this invention, conservative amino acid substitutions are defined to result from exchange of amino acids residues from within one of the following classes of residues: Class I: Ala, Gly, Ser, Thr, and Pro (representing small aliphatic side chains and hydroxyl group side chains); Class II: Cys, Ser, Thr and Tyr (representing side chains including an -OH or -SH group); Class III: Glu, Asp, Asn and Gln (carboxyl group containing side chains): Class IV: His, Arg and Lys (representing basic side chains); Class V: Ile, Val, Leu, Phe and Met (representing hydrophobic side chains); and Class VI: Phe, Trp, Tyr and His (representing aromatic side chains). The classes also include related amino acids such as 3Hyp and 4Hyp in Class I; homocysteine in Class II; 2-aminoadipic acid, 2-aminopimelic acid, γ-carboxyglutamic acid, β-carboxyaspartic acid, and the corresponding amino acid amides in Class III; ornithine, homoarginine, N-methyl lysine, dimethyl lysine, trimethyl lysine, 2,3-diaminopropionic acid, 2,4-diaminobutyric acid, homoarginine, sarcosine and hydroxylysine in Class IV; substituted phenylalanines, norleucine, norvaline, 2-aminooctanoic acid, 2-aminoheptanoic acid, statine and β-valine in Class V; and naphthylalanines, substituted phenylalanines, tetrahydroisoquinoline-3-carboxylic acid, and halogenated tyrosines in Class VI.

Preferably, the active analogs of colostrinin and its constituent peptides include polypeptides having a relatively large number of proline residues. Because proline is not a common amino acid, a "large number" preferably means that a polypeptide includes at least about 15% proline (by number), and more preferably at least about 20% proline (by number). Most preferably, active analogs include more proline residues than any other amino acid.

As stated above, active analogs of colostrinin and its constituent peptides include polypeptides having structural similarity. Structural similarity is generally determined by aligning the residues of the two amino acid sequences to optimize the number of identical amino acids along the lengths of their sequences; gaps in either or both sequences are permitted in making the alignment in order to optimize the number of identical amino acids, although the amino acids in each sequence must nonetheless remain in their proper order. Preferably, two amino acid sequences are compared using the Blastp program, version 2.0.9, of the BLAST 2 search algorithm, available at http://www.ncbi.nlm.nih.gov/gorf/bl2.html. Preferably, the default values for all BLAST 2 search parameters are used, including matrix = BLOSUM62; open gap penalty = 11, extension gap penalty = 1, gap x_dropoff = 50, expect = 10, wordsize = 3, and filter on. In the comparison of two amino acid sequences using the BLAST search algorithm, structural similarity is referred to as "identity." Preferably, an active analog of colostrinin or its constituent peptides has a structural similarity to colostrinin or one or more of its constituent peptides (preferably, one of SEQ ID NOs:1-34) of at least about 70% identity, more preferably, at least about 80% identity, and most preferably, at least about 90% identity.

Colostrinin or any combination of its peptide components or active analogs thereof can be derived (preferably, isolated and purified) naturally such as by extraction from colostrum or can be synthetically constructed using known peptide polymerization techniques. For example, the peptides of the invention may be synthesized by the solid phase method using standard methods based on either t-butyloxycarbonyl (BOC) or 9-fluorenylmethoxy-carbonyl (FMOC) protecting groups. This methodology is described by G.B. Fields et al. in Synthetic Peptides: A User's Guide, W.M. Freeman & Company, New York, NY, pp. 77-183 (1992). Moreover, gene sequence encoding the colostrinin peptides or analogs thereof can be constructed by known techniques such as expression vectors or plasmids and transfected into suitable microorganisms that will express the DNA sequences thus preparing the peptide for later extraction from the medium in which the microorganism are grown. For example, U.S. Patent No. 5,595,887 describes methods of forming a variety of relatively small peptides through expression of a recombinant gene construct coding for a fusion protein which includes a binding protein and one or more copies of the desired target peptide. After expression, the fusion protein is isolated and cleaved using chemical and/or enzymatic methods to produce the desired target peptide.

The peptides used in the methods of the present invention may be employed in a monovalent state (i.e., free peptide or a single peptide fragment coupled to a carrier molecule). The peptides may also be employed as conjugates having more than one (same or different) peptide fragment bound to a single carrier molecule. The carrier may be a biological carrier molecule (e.g., a glycosaminoglycan, a proteoglycan, albumin or the like) or a synthetic polymer (e.g., a polyalkyleneglycol or a synthetic chromatography support). Typically, ovalbumin, human serum albumin, other proteins, polyethylene glycol, or the like are employed as the carrier. Such modifications may increase the apparent affinity and/or change the stability of a peptide. The number of peptide fragments associated with or bound to each carrier can vary, but from about 4 to 8 peptides per carrier molecule are typically obtained under standard coupling conditions.

For instance, peptide/carrier molecule conjugates may be prepared by treating a mixture of peptides and carrier molecules with a coupling agent, such as a carbodiimide. The coupling agent may activate a carboxyl group on either the peptide or the carrier molecule so that the carboxyl group can react with a nucleophile (e.g., an amino or hydroxyl group) on the other member of the peptide/carrier molecule, resulting in the covalent linkage of the peptide and the carrier molecule. For example, conjugates of a peptide coupled to ovalbumin may be prepared by dissolving equal amounts of lyophilized peptide and ovalbumin in a small volume of water. In a second tube, 1-ethyl-3-(3-dimethylamino-propyl)-carboiimide hydrochloride (EDC; ten times the amount of peptide) is dissolved in a small amount of water. The EDC solution was added to the peptide/ovalbumin mixture and allowed to react for a number of hours. The mixture may then dialyzed (e.g., into phosphate buffered saline) to obtain a purified solution of peptide/ovalbumin conjugate. Peptide/carrier molecule conjugates prepared by this method typically contain about 4 to 5 peptides per ovalbumin molecule.

The present invention also provides a composition that includes one or more active agents (i.e., colostrinin, at least one constituent peptide thereof, or active analog thereof) of the invention and one or more carriers, preferably a pharmaceutically acceptable carrier. The methods of the invention include administering to, or applying to the skin of, a patient, preferably a mammal, and more preferably a human, a composition of the invention in an amount effective to produce the desired effect. The active agents of the present invention are formulated for enteral administration (oral, rectal, *etc.)* or parenteral administration (injection, internal pump, *etc.).* The administration can be via direct injection into tissue, interarterial injection, intervenous injection, or other internal administration procedures, such as through the use of an implanted pump, or via contacting the composition with a mucus membrane in a carrier designed to facilitate transmission of the composition across the mucus membrane such as a suppository, eye drops, inhaler, or other similar administration method or via oral administration in the form of a syrup, a liquid, a pill, capsule, gel coated tablet, or other similar oral administration method. The active agents can be incorporated into an adhesive plaster, a patch, a gum, and the like, or it can be encapsulated or incorporated into a bio-erodible matrix for controlled release.

The carriers for internal administration can be any carriers commonly used to facilitate the internal administration of compositions such as plasma, sterile saline solution, IV solutions or the like. Carriers for administration through mucus membranes can be any well-known in the art. Carriers for administration oral can be any carrier well-known in the art.

The formulations may be conveniently presented in unit dosage form and may be prepared by any of the methods well known in the art of pharmacy. All methods include the step of bringing the active agent into association with a carrier which constitutes one or more accessory ingredients. In general, the formulations are prepared by uniformly and intimately bringing the active agent into association with a liquid carrier, a finely divided solid carrier, or both, and then, if necessary, shaping the product into the desired formulations.

Formulations suitable for parenteral administration conveniently include a sterile aqueous preparation of the active agent, or dispersions of sterile powders of the active agent, which are preferably isotonic with the blood of the recipient. Isotonic agents that can be included in the liquid preparation include sugars, buffers, and sodium chloride. Solutions of the active agent can be prepared in water, optionally mixed with a nontoxic surfactant. Dispersions of the active agent can be prepared in water, ethanol, a polyol (such as glycerol, propylene glycol, liquid polyethylene glycols, and the like), vegetable oils, glycerol esters, and mixtures thereof. The ultimate dosage form is sterile, fluid, and stable under the conditions of manufacture and storage. The necessary fluidity can be achieved, for example, by using liposomes, by employing the appropriate particle size in the case of dispersions, or by using surfactants. Sterilization of a liquid preparation can be achieved by any convenient method that preserves the bioactivity of the active agent, preferably by filter sterilization. Preferred methods for preparing powders include vacuum drying and freeze drying of the sterile injectible solutions. Subsequent microbial contamination can be prevented using various antimicrobial agents, for example, antibacterial, antiviral and antifungal agents including parabens, chlorobutanol, phenol, sorbic acid, thimerosal, and the like. Absorption of the active agents over a prolonged period can be achieved by including agents for delaying, for example, aluminum monostearate and gelatin.

Formulations of the present invention suitable for oral administration may be presented as discrete units such as tablets, troches, capsules, lozenges, wafers, or cachets, each containing a predetermined amount of the active agent as a powder or granules, as liposomes containing the active agent, or as a solution or suspension in an aqueous liquor or non-aqueous liquid such as a syrup, an elixir, an emulsion, or a draught. The amount of active agent is such that the dosage level will be effective to produce the desired result in the subject.

Nasal spray formulations include purified aqueous solutions of the active agent with preservative agents and isotonic agents. Such formulations are preferably adjusted to a pH and isotonic state compatible with the nasal mucous membranes. Formulations for rectal or vaginal administration may be presented as a suppository with a suitable carrier such as cocoa butter, or hydrogenated fats or hydrogenated fatty carboxylic acids. Ophthalmic formulations are prepared by a similar method to the nasal spray, except that the pH and isotonic factors are preferably adjusted to match that of the eye. Topical formulations include the active agent dissolved or suspended in one or more media such as mineral oil, DMSO, polyhydroxy alcohols, or other bases used for topical pharmaceutical formulations.

Useful dosages of the active agents can be determined by comparing their *in vitro* activity and the *in vivo* activity in animal models. Methods for extrapolation of effective dosages in mice, and other animals, to humans are known in the art; for example, see U.S. Patent No. 4,938,949.

The tablets, troches, pills, capsules, and the like may also contain one or more of the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, fructose, lactose or aspartame; and a natural or artificial flavoring agent. When the unit dosage form is a capsule, it may further contain a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac, or sugar and the like. A syrup or elixir may contain one or more of a sweetening agent, a preservative such as methyl- or propylparaben, an agent to retard crystallization of the sugar, an agent to increase the solubility of any other ingredient, such as a polyhydric alcohol, for example glycerol or sorbitol, a dye, and flavoring agent. The material used in preparing any unit dosage form is substantially nontoxic in the amounts employed. The active agent may be incorporated into sustained-release preparations and devices.

### Examples

The invention will be further described by reference to the following detailed examples. The examples are meant to provide illustration and should not be construed as limiting the scope of the present invention.

### MATERIALS AND METHODS

### Preparation of Peptides:

1. Wash pre-loaded resin with DMF (dimethylformamide), then drain completely.
2. Add 10 ml of 20% piperidine/DMF to resin. Shake for 5 minutes, then drain.
3. Add another 10 ml of 20% piperidine/DMF. Shake for 30 minutes.
4. Drain reaction vessel and wash resin with DMF four times. Then wash once with DCM (dichloromethanol). Check beads using the ninhydrin test - the beads should be blue.
5. The coupling step was carried out as follows:
   a. Prepare the following solution: 1 mmole Fmoc (i.e. fluorenylmethyloxycarbonyl) amino acid 2.1 ml of 0.45 M HBTU/HOBT (1 mmol) (2-(1H-benzotriazol-1-yl)-1,1,3,3-tetramethyluronium hexafluorophosphate/N-hydroxybenzotriazole-H₂O) 348 µl of DIEA (2 mmol) (diisopropylethylamine); and
   b. Add the solution to the resin and shake for a minimum of 30 minutes.
6. Drain reaction vessel and wash the resin again with DMF four times and with DCM once.
7. Perform the ninhydrin test: If positive (no colour)-proceed to step 2 and continue synthesis; If negative (blue colour) - return to step 5 and recouple the same Fmoc amino acid.
8. After the synthesis was complete, the peptide was cleaved from the resin with 5% H₂O, 5% phenol, 3% Thionisole, 3% EDT (ethanedithiol), 3% triisopropylsilane and 81% TFA for 2 hours.
9. After 2 hours, filter into cold MTBE (methyl t-butyl ether). The precipitated peptide was then washed twice with cold MTBE and dried under nitrogen gas.
10. The molecular weight of the synthesised peptides was checked by Matrix-Assisted Laser Desorption Time-of-Flight Mass Spectroscopy (LDMS), and the purity was checked by HPLC using a C-18, 300 Angstrom, 5 µm column.

**Cells:** PC12 cell line derived from medullary pheochromocytoma cells were used to undertake studies described bellow. PC 12 cells were obtained from the American Type Culture Collection and maintained in RPMI-1640 supplemented with 10% fetal bovine serum (HYCLONE Inc), penicillin (100 U/ml) and streptomycin (100 µg/ml).

**Methods:** To evaluate the effect of colostrum, colostrinin and its component peptides on cell differentiation 3 x 10⁴ logarithmically replicating (70% confluence) PC 12 cells were seeded in 24 x well plates and cells were allowed to adhere and grow for 24 hours. Serum containing media were aspirated and replaced for serum-free RPMI containing appropriate amount of antibiotics. In four parallel, increasing concentrations (0.1, 1, 10 and 100 µg per ml) of colostrum, colostrinin and its component peptides were added directly into the media and incubated at 37°C. As a positive control, nerve growth factor (NGF) 7S (Gibco-BRL) was used at 100 ng per ml concentration (Chao et al., Cell, 68, 995-997 (1992); and Marshall et al., Cell, 80, 179-185 (1995)). Phorbol 12-myristate 13-acetate (TPA: 10 ng per ml) was used as a negative control. Eight hours later the media were changed and RPMI-1640 was added containing 1% or 10% fetal bovine serum. The cultures were microscopically investigated 2^{nd}, 4^{th} and 6^{th} days after treatment. Six days after treatment cells were fixed with paraformaldehyde (4%) and stained with 0.01% crystal violet solution. Excess of dyes were removed by ethanol washing. The final evaluation took place using a microscopy (*Axiophot2* Zeiss Inc., Germany).

### RESULTS

Control non-treated cells demonstrated the usual rounded morphology, continued to replicate and reach 70% to 80% confluency during 7 days experimental period. The mock-treated control and TPA exposed cells showed a low background level of differentiation (less than 0.01 %). In the assay system used herein, NGF (100 ng per ml) mediated a cell cycle arrest as previously described (Chao et al., Cell, 68, 995-997 (1992)). In several experiments, in the presence of 10% fetal bovine serum NGF-mediated an induction of cell differentiation that observed in 45±11% of cells. When cells were subjected to NGF-mediated differentiation in the presence of 1% serum 5% to 10% of cells showed morphological changes. The differentiated cells showed typical neuron-like morphology. In parallel experiments, nine component peptides, colostrinin and colostrum were tested. The results are summarized in Table 1.

In the presence of 1% fetal bovine serum there was no cell differentiation observed. These data indicate that some of the serum factor(s) are required to biological effect of these compounds. On the other hand, in the presence of 10% serum, the component peptides, colostrinin as well as colostrum have induced cell differentiation in PC 12 cells. The morphological changes (fibroblast-like, epitheloid, neuron-like) are shown in Figure 1. These data are in agreement with cytokine inducing activity of these peptides. For example, IFN-gamma and nerve growth factor was shown to induce similar signal transduction cascades (Peunova et al., Nature, 375, 68-73 (1995)).

**Table 1. Effect of colostrum, colostrinin, and its component peptides on morphology (differentiation) of medullary pheochromocytoma (PC 12) cells.**

| Peptide | Concentration µg/ml | 1% FBS | 10% FBS | Cell morphology |
|---|---|---|---|---|
| SEQ ID NO:1 | 100 | - | +/- | |
| | 10 | - | ++ | epitheloid |
| | 1.0 | - | + | neuron-like |
| | 0.1 | - | +/- | |
| SEQ ID NO:7 | 100 | - | +/- | |
| | 10 | - | + | fibroblast-like |
| | 1.0 | - | + | neuron-like |
| | 0.1 | - | - | |
| SEQ ID NO:8 | 100 | - | - | |
| | 10 | - | + | fibroblast-like |
| | 1.0 | - | ++ | neuron-like |
| | 0.1 | - | - | |
| SEQ ID NO:3 | 100 | - | + | |
| | 10 | - | + | fibroblast-like |
| | 1.0 | - | +/- | neuron-like |
| | 0.1 | - | - | |
| SEQ ID NO:2 | 100 | - | + | |
| | 10 | + | ++ | fibroblast-like |
| | 1.0 | - | ++ | neuron-like |
| | 0.1 | - | +/- | |
| SEQ ID NO:4 | 100 | - | ++ | fibroblast-like |
| | 10 | - | ++ | epitheloid |
| | 1.0 | - | + | neuron-like |
| | 0.1 | - | - | |
| SEQ ID NO:5 | 100 | - | + | fibroblast-like |
| | 10 | - | +/- | epitheloid |
| | 1.0 | - | - | neuron-like |
| | 0.1 | - | - | |
| SEQ ID NO:6* | 100 | - | + | |
| | 10 | - | + | fibroblast-like |
| | 1.0 | - | +/- | epitheloid |
| | 0.1 | - | - | |
| SEQ ID NO:31 | 100 | NT | NT | |
| | 10 | - | +/- | fibroblast-like |
| | 1.0 | - | | neuron-like |
| | 0.1 | | - | |
| Colostrinin | 100 | NT | NT | |
| | 10 | + | ++ | fibroblast-like |
| | 1.0 | - | ++ | neuron-like |
| | 0.1 | | - | |
| Colostrum | 100 | NT | NT | |
| | 10 | - | ++ | fibroblast-like |
| | 1.0 | - | ++ | neuron -like |
| | 0.1 | - | +/- | |
| NGF | 0.1 | ++++ | ++++ | neuron-like |
| TPA (Negative Control) | 0.1 | | | rounded |
| Control | - | - | - | rounded |

| | | | | |
|---|---|---|---|---|
| NT = Not Tested; - = ≤ 0.01% (back-ground); + = 1%; ++ = 1-5%; +++ = 6-15%; ++++ = >15% * Although the cells treated with SEQ ID NO:6 did not show neuron-like cell morphology upon visual inspection, additional tests are needed to definitively prove such morphology changes did not occur. | | | | |

Although the invention has been disclosed with reference to its preferred embodiments, from reading this description those of skill in the art may appreciate changes and modification that may be made.

### Sequence Listing Free Text

The following are all synthetic peptide sequences.

| | |
|---|---|
| SEQ ID NO:1 | MQPPPLP |
| SEQ ID NO:2 | LQTPQPLLQVMMEPQGD |
| SEQ ID NO:3 | DQPPDVEKPDLQPFQVQS |
| SEQ ID NO:4 | LFFFLPVVNVLP |
| SEQ ID NO:5 | DLEMPVLPVEPFPFV |
| SEQ ID NO:6 | MPQNFYKLPQM |
| SEQ ID NO:7 | VLEMKFPPPPQETVT |
| SEQ ID NO:8 | LKPFPKLKVEVFPFP |
| SEQ ID NO:9 | VVMEV |
| SEQ ID NO:10 | SEQP |
| SEQ ID NO:11 | DKE |
| SEQ ID NO: 12 | FPPPK |
| SEQ ID NO: 13 | DSQPPV |
| SEQ ID NO: 14 | DPPPPQS |
| SEQ ID NO: 15 | SEEMP |
| SEQ ID NO:16 | KYKLQPE |
| SEQ ID NO: 17 | VLPPNVG |
| SEQ ID NO: 18 | VYPFTGPIPN |
| SEQ ID NO: 19 | SLPQNILPL |
| SEQ ID NO:20 | TQTPWVPPF |
| SEQ ID NO:21 | LQPEIMGVPKVKETMVPK |
| SEQ ID NO:22 | HKEMPFPKYPVEPFTESQ |
| SEQ ID NO:23 | SLTLTDVEKLHLPLPLVQ |
| SEQ ID NO:24 | SWMHQPP |
| SEQ ID NO:25 | QPLPPTVMFP |
| SEQ ID NO:26 | PQSVLS |
| SEQ ID NO:27 | LSQPKVLPVPQKAVPQRDMPIQ |
| SEQ ID NO:28 | AFLLYQE |
| SEQ ID NO:29 | RGPFPILV |
| SEQ ID NO:30 | ATFNRYQDDHGEEILKSL |
| SEQ ID NO:31 | VESYVPLFP |
| SEQ ID NO:32 | FLLYQEPVLGPVR |
| SEQ ID NO:33 | LNF |
| SEQ ID NO:34 | MHQPPQPLPPTVMFP |

## Claims

1. A method for promoting in vitro cell differentiation, the method comprising contacting cells with a neuronal cell regulator selected from the group of colostrinin, a constituent peptide of colostrinin, an active analog of a constituent peptide of colostrinin, and combinations thereof, under conditions effective to change the cells in morphology to form neuronal cells,
wherein the constituent peptide of colostrinin is selected from the group of SEQ ID NO: 1 through SEQ ID NO:34. and
wherein the active analog comprises a peptide having an amino acid sequence with at least 15 percent proline and having at least 70 percent sequence identity to one or more constituent peptides of colostrinin, which are selected from the group of SEQ ID NO:1 through SEQ ID NO:34.

2. The method according to claim 1 wherein the cells are present in a cell culture, an organ, or a tissue.

3. The method according to claim 1 wherein the cells are mammalian cells,

4. The method according to claim 3 wherein the cells are human cells.

5. The method according to claim 1 wherein the cells are pluripotent cells.

6. The method according to claim 1 wherein the neuronal cell regulator is a constituent peptide of colostrinin.

7. The method of claim 6 wherein the constituent peptide of colostrinin is selected from the group of MQPPPLP (SEQ ID NO:1). LQTPQPLLQVMMEPQGD (SEQ ID NO:2), DQPPDVEKPDLQPFQVQS (SEQ ID NO:3), LFFFLPVVNVLP (SEQ ID NO:4), DLEMPVLPVEPFPFV (SEQ ID NO:5), MPQNFYKLPQM (SEQ ID NO:6), VLEMKFPPPPQETVT (SEQ ID NO:7), LKPFPKLKVEVFPFP (SEQ ID NO:8), VVMEV (SEQ ID NO:9), SEQP (SEQ ID NO:10), DKE (SEQ ID NO:11), FPPPK (SEQ ID NO:12), DSQPPV (SEQ ID NO: 13), DPPPPQS (SEQ ID NO:14), SEEMP (SEQ ID NO:15), KYKLQPE (SEQ ID NO:16), VLPPNVG (SEQ ID NO:17), VYPFTGPIPN (SEQ ID NO:18), SLPQNILPL (SEQ ID NO:19), TQTPVVVPPF (SEQ ID NO:20), LQPEIMGVPKVKETMVPK (SEQ ID NO:21), HKEMPFPKYPVEPFTESQ (SEQ ID NO:22), SLTLTDVEKLHLPLPLVQ (SEQ ID NO:23), SWMHQPP (SEQ ID NO:24), QPLPPTVMFP (SEQ ID NO:25), PQSVLS (SEQ ID NO:26), LSQPKVLPVPQKAVPQRDMPIQ (SEQ ID NO:27), AFLLYQE (SEQ ID NO:28), RGPFPILV (SEQ ID NO:29), ATFNRYQDDHGEEILKSL (SEQ ID NO:30), VESYVPLFP (SEQ ID NO:31), FLLYQEPVLGPVR (SEQ ID NO:32), LNF (SEQ ID NO:33), and MHQPPQPLPPTVMFP (SEQ ID NO:34), and combinations thereof.

8. The method according to claim 7 wherein the constituent peptide of colostrinin is selected from the group of MQPPPLP (SEQ ID NO:1), LQTPQPLLQVMMEPQGD (SEQ ID NO:2), DQPPDVEKPDLQPFQVQS (SEQ ID NO:3), LFFFLPVVNVLP (SEQ ID NO:4), DLEMPVLPVEPFPFV (SEQ ID NO:5), MPQNFYKLPQM (SEQ ID NO:6), VLEMKFPPPPQETVT (SEQ ID NO:7), LKPFPKLKVEVFPFP (SEQ ID NO:8), and combinations thereof.

9. The use of a neuronal cell regulator selected from the group of colostrinin, a constituent peptide of colostrinin, an active analog of a constituent peptide of colostrinin, and combinations thereof,
wherein the constituent peptide of colostrinin is selected from the group of SEQ ID NO:1 through SEQ ID NO:34, and
wherein the active analog comprises a peptide having an amino acid sequence with at least 15 percent proline and having at least 70 percent sequence identity to one or more constituent peptides of colostrinin, which are selected from the group of SEQ ID NO:1 through SEQ ID NO:34,
in the manufacture of a medicament for promoting neuronal cell differentiation in a patient.

10. The use according to claim 9 wherein the patient is a human.

11. The use according to claim 9 wherein the neuronal cell regulator is a constituent peptide of colostrinin.

12. The use according to claim 11 wherein the constituent peptide of colostrinin is selected from the group of MQPPPLP (SEQ ID NO:1), LQTPQPLLQVMMEPQGD (SEQ ID NO:2), DQPPDVEKPDLQPFQVQS (SEQ ID NO:3), LFFFLPVVNVLP (SEQ ID NO:4), DLEMPVLPVEPFPFV (SEQ ID NO:5), MPQNFYKLPQM (SEQ ID NO:6), VLEMKFPPPPQETVT (SEQ ID NO:7), LKPFPKLKVEVFPFP (SEQ ID NO:8), VVMEV (SEQ ID NO:9), SEQP (SEQ ID NO:10), DKE (SEQ ID NO: 11), FPPPK (SEQ ID NO: 12), DSQPPV (SEQ ID NO: 13), DPPPPQS (SEQ ID NO: 14), SEEMP (SEQ ID NO: 15). KYKLQPE (SEQ ID NO:16), VLPPNVG (SEQ ID NO:17), VYPFTGPIPN (SEQ ID NO:18), SLPQNILPL (SEQ ID NO:19), TQTPVVVPPF (SEQ ID NO:20), LQPEIMGVPKVKETMVPK (SEQ ID NO:21), HKEMPFPKYPVEPFTESQ (SEQ ID NO:22), SLTLTDVEKLHLPLPLVQ (SEQ ID NO:23), SWMHQPP (SEQ ID NO:24), QPLPPTVMFP (SEQ ID NO:25). PQSVLS (SEQ ID NO:26), LSQPKVLPVPQKAVPQRDMPIQ (SEQ ID NO:27), AFLLYQE (SEQ ID NO:28), RGPFPILV (SEQ ID NO:29), ATFNRYQDDHGEEILKSL (SEQ ID NO:30), VESYVPLFP (SEQ ID NO:31), FLLYQEPVLGPVR (SEQ ID NO:32). LNF (SEQ ID NO:33), and MHQPPQPLPPTVMFP (SEQ ID NO:34), and combinations thereof.

13. The use according to claim 12 wherein the constituent peptide of colostrinin is selected from the group of MQPPPLP (SEQ ID NO:1), LQTPQPLLQVMMEPQGD (SEQ ID NO:2), DQPPDVEKPDLQPFQVQS (SEQ ID NO.3), LFFFLPVVNVLP (SEQ ID NO:4), DLEMPVLPVEPFPFV (SEQ ID NO:5), MPQNFYKLPQM (SEQ ID NO:6), VLEMKFPPPPQETVT (SEQ ID NO:7). LKPFPKLKVEVFPFP (SEQ ID NO:8), and combinations thereof.

14. An in vitro method for treating damaged neuronal cells, the method comprising contacting nonfunctional neuronal cells with a neuronal cell regulator selected from the group of colostrinin, a constituent peptide of colostrinin, an active analog of a constituent peptide of colostrinin, and combinations thereof, under conditions effective to convert the damaged neuronal cells to functional neuronal cells;
wherein the constituent peptide of colostrinin is selected from the group of SEQ ID NO:1 through SEQ ID NO:34:
wherein the active analog comprises a peptide having an amino acid sequence with at least 15 percent proline and having at least 70 percent sequence identity to one or more constituent peptides of colostrinin, which are selected from the group of SEQ ID No:1 through SEQ ID NO:34, and
wherein the nonfunction is the result of neurodegeneration.

15. The use of a neuronal cell regulator selected from the group of colostrinin, a constituent peptide of colostrinin, an active analog of a constituent peptide of colostrinin, and combinations thereof;
wherein the constituent peptide of colostrinin is selected from the group of SEQ ID NO:1 through SEQ ID NO:34: and
wherein the active analog comprises a peptide having an amino acid sequence with at least 15 percent proline and having at least 70 percent sequence identity to one or more constituent peptides of colostrinin, which are selected from the group of SEQ ID NO:1 through SEQ ID NO:34,
in the manufacture of a medicament for treating damaged neuronal cells in a patient.

16. A method for promoting in vitro neuronal cell differentiation, the method comprising contacting pluripotent cells of the nervous system with a neuronal cell regulator selected from the group of colostrinin, a constituent peptide of colostrinin, an active analog of a constituent peptide of colostrinin, and combinations thereof, under conditions effective to change the pluripotent cells of the nervous system in morphology to form neuronal cells;
wherein the constituent peptide of coloctrinin is selected from the group of SEQ ID NO: 1 through SEQ ID NO:34; and
wherein the active analog comprises a peptide having an amino acid sequence with at least 15 percent proline and having at least 70 percent sequence identity to one or more constituent peptides of colostrinin, which are selected from the group of SEQ ID NO:1 through SEQ ID NO:34.

17. The use of a neuronal cell regulator selected from the group of colostrinin, a constituent peptide of colostrinin, an active analog of a constituent peptide of colostrinin, and combinations thereof;
wherein the constituent peptide of colostrinin is selected from the group of SEQ ID NO: 1 through SEQ ID NO:34; and
wherein the active analog comprises a peptide having an amino acid sequence with at least 15 percent proline and having at least 70 percent sequence identity to one or more constituent peptides of colostrinin, which are selected from the group of SEQ ID NO:1 through SEQ ID NO:34, in the manufacture of a medicament for promoting differentiation of pluripotent cells of the nervous system to form neuronal cells in a patient.

## Patentansprüche

1. Verfahren zum Fördern der In-vitro-Zelldifferenzierung, wobei das Verfahren das Inkontaktbringen der Zellen mit einem Regulator neuronaler Zellen umfasst, der ausgewählt ist aus der Gruppe bestehend aus Colostrinin, einem Peptidbestandteil von Colostrinin, einem aktiven Analogon eines Peptidbestandteils von Colostrinin und Kombinationen davon, unter Bedingungen, die wirksam sind, um die Morphologie der Zellen zu verändern, um neuronale Zellen zu bilden,
worin der Peptidbestandteil von Colostrinin ausgewählt ist aus der Gruppe bestehend aus SEQ ID NR:1 bis SEQ ID NR:34 und
worin das aktive Analogon ein Peptid umfasst, das eine Aminosäuresequenz mit wenigstens 15% Prolin hat und das wenigstens 70 % Sequenzidentiät mit einem oder mehreren Peptidbestandteil(en) von Colostrinin aufweist, die ausgewählt sind aus der Gruppe und bestehend aus SEQ ID NR:1 bis SEQ ID NR:34.

2. Das Verfahren gemäß Anspruch 1, worin die Zellen in einer Zellkultur, einem Organ oder einem Gewebe vorhanden sind.

3. Das Verfahren gemäß Anspruch 1, worin die Zellen Säugerzellen sind.

4. Das Verfahren gemäß Anspruch 3, worin die Zellen humane Zellen sind.

5. Das Verfahren gemäß Anspruch 1, worin die Zellen pluripotente Zellen sind.

6. Das Verfahren gemäß Anspruch 1, worin der Regulator neuronaler Zellen ein Peptidbestandteil von Colostrinin ist.

7. Das Verfahren gemäß Anspruch 6, worin der Peptidbestandteil von Colostrinin ausgewählt ist aus der Gruppe bestehend aus MQPPPLP (SEQ ID NR:1), LQTPQPLLQVMMEPQGD (SEQ ID NR:2), DQPPDVEKPDLQPFQVQS (SEQ ID NR:3), LFFFLPVVNVLP (SEQ ID NR:4), DLEMPVLPVEPFPFV (SEQ ID NR:5), MPQNFYKLPQM (SEQ ID NR:6), VLEMKFPPPPQETVT (SEQ ID NR:7), LKPFPKLKVEVFPFP (SEQ ID NR:8), VVMEV (SEQ ID NR:9), SEQP (SEQ ID NR:10), DKE (SEQ ID NR:11), FPPPK (SEQ ID NR:12), DSQPPV (SEQ ID NR:13), DPPPPQS (SEQ ID NR:14), SEEMP (SEQ ID NR:15), KYKLQPE (SEQ ID NR:16), VLPPNVG (SEQ ID NR:17), VYPFTGPIPN (SEQ ID NR:18), SLPQNILPL (SEQ ID NR:19), TQTPVWPPF (SEQ ID NR:20), LQPEIMGVPKVKETMVPK (SEQ ID NR:21), HKEMPFPKYPVEPFTESQ (SEQ ID NR:22), SLTLTDVEKLHLPLPLVQ (SEQ ID NR:23), SWMHQPP (SEQ ID NR:24), QPLPPTVMFP (SEQ ID NR:25), PQSVLS (SEQ ID NR:26), LSQPKVLPVQKAVPQRDMPIQ (SEQ ID NR:27), AFLLYQE (SEQ ID NR:28), RGPFPILV (SEQ ID NR:29), ATFNRYQDDHGEEILKSL (SEQ ID NR:30), VESYVPLFP (SEQ ID NR:31), FLLYQEPVLGPVR (SEQ ID NR:32), LNF (SEQ ID NR:33) und MHQPPQPLPPTVMFP (SEQ ID NR:34), sowie Kombinationen davon.

8. Das Verfahren gemäß Anspruch 7, worin der Peptidbestandteil von Colostrinin ausgewählt ist aus der Gruppe bestehend aus MQPPPLP (SEQ ID NR:1), LQTPQPLLQVMMEPQGD (SEQ ID NR:2), DQPPDVEKPDLQPFQVQS (SEQ ID NR:3), LFFFLPVVNVLP (SEQ ID NR:4), DLEMPVLPVEPFPFV (SEQ ID NR:5), MPQNFYKLPQM (SEQ ID NR:6), VLEMKFPPPPQETVT (SEQ ID NR:7), LKPFPKLKVEVFPFP (SEQ ID NR:8), sowie Kombinationen davon.

9. Verwendung eines Regulators neuronaler Zellen, der ausgewählt ist aus der Gruppe bestehend aus Colostrinin, einem Peptidbestandteil von Colostrinin, einem aktiven Analogon eines Peptidbestandteils von Colostrinin und Kombinationen davon,
worin der Peptidbestandteil von Colostrinin ausgewählt ist aus der Gruppe bestehend aus SEQ ID NR:1 bis SEQ ID NR:34 und
worin das aktive Analogon ein Peptid umfasst, das eine Aminosäuresequenz mit wenigstens 15% Prolin hat und das wenigstens 70% Sequenzidentität mit einem oder mehreren Peptidbestandteil(en) von Colostrinin aufweist, die ausgewählt sind aus der Gruppe bestehend aus SEQ ID NR:1 bis SEQ ID NR:34,
bei der Herstellung eines Medikamentes zum Fördern der neuronalen Zelldifferenzierung in einem Patienten.

10. Die Verwendung gemäß Anspruch 9, worin der Patient ein Mensch ist.

11. Die Verwendung gemäß Anspruch 9, worin der Regulator neuronaler Zellen ein Peptidbestandteil von Colostrinin ist.

12. Die Verwendung gemäß Anspruch 11, worin der Peptid bestandteil von Colostrinin ausgewählt ist aus der Gruppe bestehen aus MQPPPLP (SEQ ID NR:1), LQTPQPLLQVMMEPQGD (SEQ ID NR:2), DQPPDVEKPDLQPFQVQS (SEQ ID NR:3), LFFFLPWNVLP (SEQ ID NR: 4), DLEMPVLPVEPFPFV (SEQ ID NR:5), MPQNFYKLPQM (SEQ ID NR:6), VLEMKFPPPPQETVT (SEQ ID NR:7), LKPFPKLKVEVFPFP (SEQ ID NR:8), WMEV (SEQ ID NR:9), SEQP (SEQ ID NR:10), DKE (SEQ ID NR:11), FPPPK (SEQ ID NR:12), DSQPPV (SEQ ID NR:13), DPPPPQS (SEQ ID NR:14), SEEMP (SEQ ID NR:15), KYKLQPE (SEQ ID NR:16), VLPPNVG (SEQ ID NR:17), VYPFTGPIPN (SEQ ID NR:18), SLPQNILPL (SEQ ID NR:19), TQTPVWPPF (SEQ ID NR:20), LQPEIMGVPKVKETMVPK (SEQ ID NR:21), HKEMPFPKYPVEPFTESQ (SEQ ID NR:22), SLTLTDVEKLHLPLPLVQ (SEQ ID NR:23), SWMHQPP (SEQ ID NR:24), QPLPPTVMFP (SEQ ID NR:25), PQSVLS (SEQ ID NR:26), LSQPKVLPVQKAVPQRDMPIQ (SEQ ID NR:27), AFLLYQE (SEQ ID NR:28), RGPFPILV (SEQ ID NR:29), ATFNRYQDDHGEEILKSL (SEQ ID NR:30), VESYVPLFP (SEQ ID NR:31), FLLYQEPVLGPVR (SEQ ID NR:32), LNF (SEQ ID NR:33) und MHQPPQPLPPTVMFP (SEQ ID NR:34), sowie Kombinationen davon.

13. Die Verwendung gemäß Anspruch 12, worin der Peptidbestandteil von Colostrinin ausgewählt ist aus der Gruppe bestehend aus MQPPPLP (SEQ ID NR:1), LQTPQPLLQVMMEPQGD (SEQ ID NR:2), DQPPDVEKPDLQPFQVQS (SEQ ID NR:3), LFFFLPWNVLP (SEQ ID NR:4), DLEMPVLPVEPFPFV (SEQ ID NR:5), MPQNFYKLPQM (SEQ ID NR:6), VLEMKFPPPPQETVT (SEQ ID NR:7), LKPFPKLKVEVFPFP (SEQ ID NR:8), sowie Kombinationen davon.

14. In-vitro-Verfahren zum Behandeln geschädigter neuronaler Zellen, wobei das Verfahren das Inkontaktbringen nicht-funktioneller neuronaler Zellen mit einem Regulator neuronaler Zellen umfasst, der ausgewählt ist aus der Gruppe bestehend aus Colostrinin, einem Peptidbestandteil von Colostrinin, einem aktiven Analogon eines Peptidestandteils von Colostrinin und Kombinationen davon, unter Bedingungen, die wirksam sind, um die geschädigten neuronalen Zellen zu funktionellen neuronalen Zellen umzuwandeln;
worin der Peptidbestandteil von Colostrinin ausgewählt ist aus der Gruppe bestehend aus SEQ ID NR:1 bis SEQ ID NR:34;
worin das aktive Analogon ein Peptid umfasst, das eine Aminosäuresequenz mit wenigstens 15% Prolin hat und das wenigstens 70% Sequenzidentiät mit einem oder mehreren Peptidbestandteil(en) von Colostrinin aufweist, die ausgewählt sind aus der Gruppe bestehend aus SEQ ID NR:1 bis SEQ ID NR:34 und
worin die Nichtfunktion das Ergebnis einer Neurodegeneration ist.

15. Verwendung eines Regulators neuronaler Zellen, der ausgewählt ist aus der Gruppe bestehen aus Colostrinin, einem Peptid bestandteil von Colostrinin, einem aktiven Analogon eines Peptid bestandteils von Colostrinin und Kombinationen davon;
worin der Peptid bestandteil von Colostrinin ausgewählt ist aus der Gruppe bestehend aus SEQ ID NR:1 bis SEQ ID NR:34 und
worin das aktive Analogon ein Peptid umfasst, das eine Aminosäuresequenz mit wenigstens 15% Prolin hat und das wenigstens 70% Sequenzidentiät mit einem oder mehreren Peptidbestandteil(en) von Colostrinin aufweist, die ausgewählt sind aus der Gruppe bestehend aus SEQ ID NR:1 bis SEQ ID NR:34,
bei der Herstellung eines Medikamentes zum Behandeln geschädigter neuronaler Zellen in einem Patienten.

16. Verfahren zum Fördern der In-vitro-Differenzierung neuronaler Zellen, wobei das Verfahren das Inkontaktbringen pluripotenter Zellen des Nervensystems mit einem Regulator neuronaler Zellen umfasst, der ausgewählt ist aus der Gruppe bestehend aus Colostrinin, einem Peptidbestandteil von Colostrinin, einem aktiven Analogon eines Peptidbestandteils von Colostrinin und Kombinationen davon, unter Bedingungen, die wirksam sind, um die pluripotenten Zellen des Nervensystems in ihrer Morphologie zu verändern, um neuronale Zellen zu bilden;
worin der Peptidbestandteil von Colostrinin ausgewählt ist aus der Gruppe bestehend aus SEQ ID NR:1 bis SEQ ID NR:34; und
worin das aktive Analogon ein Peptid umfasst, das eine Aminosäuresequenz mit wenigstens 15% Prolin hat und das wenigstens 70% Sequenzidentität mit einem oder mehreren Peptidbestandteil(en) von Colostrinin aufweist, die ausgewählt sind aus der Gruppe bestehend aus SEQ ID NR:1 bis SEQ ID NR:34.

17. Verwendung eines Regulators neuronaler Zellen, der ausgewählt ist aus der Gruppe bestehen aus Colostrinin, einem Peptidbestandteil von Colostrinin, einem aktiven Analogon eines Peptidbestandteils von Colostrinin und Kombinationen davon;
worin der Peptidbestandteil von Colostrinin ausgewählt ist aus der Gruppe bestehend aus SEQ ID NR:1 bis SEQ ID NR:34; und
worin das aktive Analogon ein Peptid umfasst, das eine Aminosäuresequenz mit wenigstens 15% Prolin hat und das wenigstens 70% Sequenzidentiät mit einem oder mehreren Peptidbestandteil(en) von Colostrinin aufweist, die ausgewählt sind aus der Gruppe bestehen aus SEQ ID NR:1 bis SEQ ID NR:34,
bei der Herstellung eines Medikamentes zum Fördern der Differenzierung pluripotenter Zellen des Nervensystems, um neuronale Zellen in einem Patienten zu bilden.

## Revendications

1. Procédé pour favoriser la différenciation cellulaire *in vitro,* le procédé comprenant la mise en contact de cellules avec un régulateur de cellule neuronale choisi dans le groupe de la colostrinine, d'un peptide constitutif de la colostrinine, d'un analogue actif d'un peptide constitutif de la colostrinine, et de leurs combinaisons, dans des conditions efficaces pour changer la morphologie des cellules pour former des cellules neuronales,
dans lequel le peptide constitutif de la colostrinine est choisi dans le groupe de SEQ ID n° 1 à SEQ ID n° 34, et
dans lequel l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 à SEQ ID n° 34.

2. Procédé selon la revendication 1, dans lequel les cellules sont présentes dans une culture cellulaire, un organe ou un tissu.

3. Procédé selon la revendication 1, dans lesquelles les cellules sont des cellules de mammifère.

4. Procédé selon la revendication 3, dans lequel les cellules sont des cellules humaines.

5. Procédé selon la revendication 1, dans lequel les cellules sont des cellules pluripotentes.

6. Procédé selon la revendication 1, dans lequel le régulateur de cellule neuronale est un peptide constitutif de la colostrinine.

7. Procédé selon la revendication 6, dans lequel le peptide constitutif de la colostrinine est choisi dans le groupe de MQPPPLP (SEQ ID NO:1), LQTPQPLLQVMMEPQGD (SEQ ID NO: 2), DQPPDVEKPDLQPFQVQS (SEQ ID NO: 3), LFFFLPVVNVLP (SEQ ID NO: 4), DLEMPVLPVEPFPFV (SEQ ID NO:5), MPQNFYKLPQM (SEQ ID NO: 6), VLEMKFPPPPQETVT (SEQ ID NO: 7), LKPFPKLKVEVFPFP (SEQ ID NO: 8), VVMEV (SEQ ID NO: 9), SEQP (SEQ ID NO: 10), DKE (SEQ ID NO:11), FPPPK (SEQ ID NO: 12), DSQPPV (SEQ ID NO: 13), DPPPPQS (SEQID NO: 14), SEEMP (SEQ ID NO: 15), KYKLQPE (SEQ ID NO: 16), VLPPNVG (SEQ ID NO: 17), VYPFTGPIPN (SEQ ID NO: 18), SLPQNILPL (SEQ ID NO: 19), TQTPVVVPPF (SEQ ID NO: 20), LQPEIMGVPKVKETMVPK (SEQ ID NO: 21), HKEMPFPKYPVEPFTESQ (SEQ ID NO: 22),SLTLTDVEKLHLPLPLVQ (SEQ ID NO: 23), SWMHQPP (SEQ ID NO: 24), QPLPPTVMFP (SEQ ID NO: 25), PQSVLS (SEQ ID NO: 26), LSQPKVLPVPQKAVPQRDMPIQ (SEQ ID NO: 27), AFLLYQE (SEQ ID NO: 28), RGPFPILV (SEQID NO: 29), ATFNRYQDDHGEEILKSL (SEQ ID NO: 30), VESYVPLFP (SEQ ID NO:31), FLLYQEPVLGPVR (SEQ ID NO: 32), LNF (SEQID NO: 33), et MHQPPQPLPPTVMFP (SEQ ID NO: 34), et de leurs combinaisons.

8. Procédé selon la revendication 7, dans lequel le peptide constitutif de la colostrinine est choisi dans le groupe de MQPPPLP (SEQ ID NO: 1), LQTPQPLLQVMMEPQGD (SEQ ID NO: 2), DQPPDVEKPDLQPFQVQS (SEQ ID NO: 3), LFFFLPVVNVLP (SEQ ID NO: 4), DLEMPVLPVEPFPFV (SEQ ID NO:5), MPQNFYKLPQM (SEQ ID NO: 6), VLEMKFPPPPQETVT (SEQID NO: 7), LKPFPKLKVEVFPFP (SEQ IDNO: 8), et de leurs combinaisons.

9. Utilisation d'un régulateur de cellule neuronale choisi dans le groupe de la colostrinine, d'un peptide constitutif de colostrinine, d'un analogue actif d'un peptide constitutif de la colostrinine, et de leurs combinaisons,
dans laquelle le peptide constitutif de la colostrinine est choisi dans le groupe de SEQ ID n° 1à SEQ ID n° 34, et
dans laquelle l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 SEQ ID n° 34,
dans la fabrication d'un médicament pour favoriser la différenciation de cellule neuronale chez un patient.

10. Utilisation selon la revendication 9, dans laquelle le patient est un humain.

11. Utilisation selon la revendication 9, dans laquelle le régulateur de cellule neuronale est un peptide constitutif de la colostrinine.

12. Utilisation selon la revendication 11, dans laquelle le peptide constitutif de la colostrinine est choisi dans le groupe de MQPPPLP (SEQ ID NO:1), LQTPQPLLQVMMEPQGD (SEQ ID NO: 2), DQPPDVEKPDLQPFQVQS (SEQ ID NO: 3), LFFFLPVVNVLP (SEQ ID NO: 4), DLEMPVLPVEPFPFV (SEQ ID NO:5), MPQNFYKLPQM (SEQ ID NO: 6), VLEMKFPPPPQETVT (SEQ ID NO: 7), LKPFPKLKVEVFPFP (SEQ ID NO: 8), VVMEV (SEQ ID NO: 9), SEQP (SEQ ID NO: 10), DKE (SEQ IDNO:11), FPPPK (SEQ ID'NO: 12), DSQPPV (SEQ ID NO: 13), DPPPPQS (SEQ ID NO: 14), SEEMP (SEQ ID NO: 15), KYKLQPE (SEQ ID NO: 16), VLPPNVG (SEQ ID NO: 17), VYPFTGPIPN (SEQ ID NO: 18), SLPQNILPL (SEQ ID NO: 19), TQTPVVVPPF (SEQ ID NO: 20), LQPEIMGVPKVKETMVPK (SEQ ID NO: 21), HKEMPFPKYPVEPFTESQ (SEQ ID NO: 22), SLTLTDVEKLHLPLPLVQ (SEQ ID NO: 23), SWMHQPP (SEQ ID NO: 24), QPLPPTVMFP (SEQ ID NO: 25), PQSVLS (SEQ ID NO: 26), LSQPKVLPVPQKAVPQRDMPIQ (SEQ ID NO: 27), AFLLYQE (SEQ ID NO: 28), RGPFPILV (SEQID NO: 29), ATFNRYQDDHGEEILKSL (SEQ ID NO: 30), VESYVPLFP (SEQ ID NO:31), FLLYQEPVLGPVR (SEQ ID NO: 32), LNF (SEQID NO: 33), et MHQPPQPLPPTVMFP (SEQ ID NO: 34), et de leurs combinaisons

13. Utilisation selon la revendication 12, dans laquelle le peptide constitutif de la colostrinine est choisi dans le groupe de MQPPPLP (SEQ ID NO: 1), LQTPQPLLQVMMEPQGD (SEQ ID NO: 2), DQPPDVEKPDLQPFQVQS (SEQ ID NO: 3), LFFFLPVVNVLP (SEQ ID NO: 4), DLEMPVLPVEPFPFV (SEQ ID NO:5), MPQNFYKLPQM (SEQ ID NO: 6), VLEMKFPPPPQETVT (SEQID NO: 7), LKPFPKLKVEVFPFP (SEQ IDNO: 8), et de leurs combinaisons.

14. Procédé *in vitro* pour traiter des cellules neuronales endommagées, le procédé comprenant la mise en contact de cellules neuronales non fonctionnelles avec un régulateur de cellule neuronale choisi dans le groupe de la colostrinine, d'un peptide constitutif de la colostrinine, d'un analogue actif d'un peptide constitutif de la colostrinine, et de leurs combinaisons, dans des conditions efficaces pour convertir les cellules neuronales endommagées en cellules neuronales fonctionnelles ;
dans lequel le peptide constitutif de la colostrinine est choisi dans le groupe de SEQ ID n° 1 à SEQ ID n° 34 ;
dans lequel l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 à SEQ ID n° 34, et
dans lequel le non fonctionnement est le résultat d'une neurodégénérescence.

15. Utilisation d'un régulateur de cellule neuronale choisi dans le groupe de la colostrinine, d'un peptide constitutif de la colostrinine, d'un analogue actif d'un peptide constitutif de la colostrinine, et de leurs combinaisons ;
dans lequel le peptide constitutif de la colostrinine est choisi dans le groupe de SEQ ID n° 1 à SEQ ID n°34 ; et
dans lequel l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 à SEQ ID n° 34,
dans la fabrication d'un médicament pour traiter les cellules neuronales endommagées chez un patient.

16. Procédé pou favoriser la différenciation cellulaire neuronale *in vitro,* le procédé comprenant la mise en contact de cellules pluripotentes du système nerveux avec un régulateur de cellule neuronale choisi dans le groupe de la colostrinine, d'un peptide constitutif de la colostrinine, et d'un analogue actif d'un peptide constitutif de la colostrinine, et de leurs combinaisons, dans des conditions efficaces pour changer la morphologie de cellules pluripotentes du système nerveux pour former des cellules neuronales ;
dans lequel le peptide constitutif de la colostrinine est choisi dans le groupe de SEQ ID n° 1 à SEQ ID n°34.
dans lequel l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 à SEQ ID n° 34.

17. Utilisation d'un régulateur de cellule neuronale choisi dans le groupe de la colostrinine, d'un peptide constitutif de la colostrinine, d'un analogue actif d'un peptide constitutif de la colostrinine, et de leurs combinaisons ;
dans laquelle le peptide constitutif de la colostrinine est choisi dans le groupe de SEQ ID n° 1 à SEQ ID n°34 ; et
dans laquelle l'analogue actif comprend un peptide ayant une séquence d'acides aminés avec au moins 15 pour cent de proline et ayant au moins 70 pour cent d'identité de séquence avec un ou plusieurs peptides constitutifs de la colostrinine, qui sont choisis dans le groupe de SEQ ID n° 1 à SEQ ID n° 34, dans la fabrication d'un médicament pour favoriser la différenciation de cellules pluripotentes du système nerveux pour former des cellules neuronales chez un patient.
